(19) Europäisches Patentamt
European Patent Office
Office européen des brevets

(11) EP 4 810 004 A1

(12) **EUROPEAN PATENT APPLICATION**
published in accordance with Art. 153(4) EPC

(43) Date of publication:
**23.09.2026 Bulletin 2026/39**

(21) Application number: **24907116.8**

(22) Date of filing: **29.11.2024**

(51) International Patent Classification (IPC):
***A61K 8/25** (2006.01)* ***A61K 33/06** (2006.01)*
***A61P 1/02** (2006.01)* ***A61Q 11/00** (2006.01)*

(52) Cooperative Patent Classification (CPC):
**A61K 8/25; A61K 33/06; A61P 1/02; A61Q 11/00**

(86) International application number:
**PCT/JP2024/042346**

(87) International publication number:
**WO 2025/134729 (26.06.2025 Gazette 2025/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **18.12.2023 JP 2023213073**

(71) Applicant: **Sunstar Inc.**
**Takatsuki-shi**
**Osaka 569-1195 (JP)**

(72) Inventors:
- **NISHIOKA Ryotaro**
**Takatsuki-shi, Osaka 569-1195 (JP)**
- **KINOSAKI Mebae**
**Takatsuki-shi, Osaka 569-1195 (JP)**
- **YABUMOTO Manami**
**Takatsuki-shi, Osaka 569-1195 (JP)**

(74) Representative: **Murgitroyd & Company**
**165-169 Scotland Street**
**Glasgow G5 8PL (GB)**

(54) **ORAL COMPOSITION AND DENTIN PROTECTIVE AGENT**

(57) An oral composition and a dentin protective agent according to the present disclosure are characterized by containing calcium silicate having an Si/Ca ratio of 1.15 to 2.3.



Processed by Luminess, 75001 PARIS (FR)

## Description

### TECHNICAL FIELD

The present disclosure relates to an oral composition and a dentin protective agent.

### BACKGROUND ART

In general, dental caries occurs when bacteria in dental plaque on the tooth surface metabolize sugars in food and drink to produce acids, and the acids demineralize hydroxyapatite in enamel and dentin.

Conventionally, for example, an oral composition disclosed in Patent Literature 1 is known. Such an oral composition contains (A) a pharmacologically acceptable zinc compound and (B) a pharmacologically acceptable aldehyde compound, etc. Such an oral composition prevents dental caries by suppressing elution of minerals from dental hard tissues and maintaining the acid resistance of enamel, dentin, and the like.

### CITATION LIST

#### Patent Literature

Patent Literature 1: JPH11-228368A

### SUMMARY OF INVENTION

#### Technical Problem

In recent years, particularly in elderly persons, increased risks of root caries on exposed dentin surfaces due to gingival recession, hypersensitivity, and the like have become problematic. Dentin erosion involves demineralization of hydroxyapatite and breakdown of collagen contained therein as an organic component, and it is difficult to prevent dentin erosion by conventional techniques for suppressing demineralization alone. Accordingly, effective protection of dentin has been desired.

#### Solution to Problem

As a result of studies conducted to solve the above problems, the present inventors have found that calcium silicate having a predetermined Si/Ca ratio exhibits an excellent dentin-protective effect.

Aspects for solving the above problems will now be described.

An oral composition according to Aspect 1 includes calcium silicate having a Si/Ca ratio of 1.15 to 2.3, inclusive.

In Aspect 2, the oral composition according to Aspect 1 is for dentin protection.

A dentin protective agent according to Aspect 3 includes calcium silicate having a Si/Ca ratio of 1.15 to 2.3, inclusive.

#### Advantageous Effects of Invention

The oral composition and dentin protective agent of the present disclosure provides an excellent dentin-protective effect.

### DESCRIPTION OF EMBODIMENTS

Embodiments of an oral composition and a dentin protective agent according to the present disclosure will now be described.

Because the oral composition and the dentin protective agent contain similar components, the dentin protective agent will now be described.

In the present disclosure, the term "oral composition" refers to a composition intended primarily for use in the oral cavity. The oral composition of the present disclosure includes not only oral preparations that are discharged from the oral cavity after use, but also ingestible foods and beverages. The dentin protective agent is not limited to one intended for use in the oral cavity.

The dentin protective agent contains calcium silicate having a Si/Ca ratio of 1.15 to 2.3, inclusive.

The components of the dentin protective agent will now be described.

<Calcium Silicate>

Calcium silicate is a generic term for substances in which calcium oxide (CaO), silicon dioxide ($SiO_2$), water, and the like are combined in various combinations, and is represented by $x(CaO)\cdot y(SiO_2)\cdot z(H_2O)$. In the dentin protective agent of the present disclosure, calcium silicate having a Si/Ca ratio within a predetermined range is used as a raw material.

The Si/Ca ratio of the calcium silicate is between 1.15 and 2.3, inclusive. The upper limit of the Si/Ca ratio of the calcium silicate is preferably 2.2; more preferably 2.1. The lower limit of the Si/Ca ratio of the calcium silicate is preferably 1.2; more preferably 1.3. The upper or lower limit value of the range may be, for example, 1.15, 1.2, 1.25, 1.3, 1.35, 1.4, 1.45, 1.5, 1.55, 1.6, 1.65, 1.7, 1.75, 1.8, 1.85, 1.9, 1.95, 2.0, 2.05, 2.1, 2.15, 2.2, 2.25, or 2.3.

The Si/Ca ratio of the calcium silicate can be measured using energy-dispersive X-ray spectroscopy (SEM-EDX). More specifically, the Si/Ca ratio of the calcium silicate used as a raw material is measured by the following method.

(1) Conductive carbon tape is attached to a sample holder, and calcium silicate is sprinkled thereon.
(2) Excess powder is removed with a blower, and elemental analysis is performed by energy-dispersive X-ray spectroscopy (SEM-EDX).
(3) Each sample is measured four times, and the average Si/Ca ratio is calculated from the numbers of Si atoms and Ca atoms.

The Si/Ca ratio of the calcium silicate can be adjusted by changing the ratio of silicon dioxide and calcium oxide constituting the molecule of calcium silicate.

The average particle size (D50) of the calcium silicate as determined by a wet laser diffraction scattering method is not particularly limited. The average particle size (D50) of the calcium silicate is, for example, between 5 μm and 35 μm, inclusive.

The upper or lower limit value of the range may be, for example, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, or 35 μm.

The degree of aggregation of the calcium silicate is not particularly limited. The degree of aggregation of the calcium silicate is, for example, between 0.5% and 15%, inclusive.

The upper or lower limit value of the range may be, for example, 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, or 15%.

The degree of aggregation of the calcium silicate is determined by the following method using sieves.

The degree of aggregation is a value obtained by placing powder on the uppermost sieve of the three sieves listed in Table 1, vibrating the sieves for a predetermined time and at a predetermined intensity, and calculating, according to the following equation, the amount of powder that has agglomerated as a result of the vibration.

When most of the measured particle sizes of the calcium silicate sample are less than or equal to the aperture size of the lowermost sieve shown below, standard sieves are used and vibrated for a predetermined time and at a predetermined intensity, and the degree of aggregation is evaluated from the amount introduced and the amount remaining on the sieve.

[Table 1]

| Average Bulk Density ($kg/m^3$) | Sieve Opening Diameter (μm) | | |
|---|---|---|---|
| | Upper | Middle | Lower |
| Less than 400 | 355 | 250 | 150 |
| 400 or more and less than 900 | 250 | 150 | 75 |
| 900 or more | 150 | 75 | 45 |

Degree of aggregation (%) = (amount remaining on the upper sieve/amount of sample introduced + amount remaining on the middle sieve/amount of sample introduced × 3/5 + amount remaining on the lower sieve/amount of sample introduced × 1/5) × 100

The content of the calcium silicate in the dentin protective agent is not particularly limited. The content of the calcium silicate is, for example, 0.1 mass % to 20 mass %, inclusive.

The upper or lower limit value of the range may be, for example, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, or 20 mass %.

<Other Components>

Depending on the intended purpose, form, use, and the like, the dentin protective agent may further contain components other than those described above, for example, medicinal ingredients, surfactants, abrasives, humectants, thickeners, stabilizers, preservatives, sweeteners, pH adjusters, antioxidants, flavoring agents, and colorants. As these components, known components commonly incorporated into dentin protective agents may be used. Each of these components may be used alone, or two or more thereof may be used in combination.

Examples of medicinal ingredients include bactericides, circulation promoters, anti-inflammatory agents, agents for ameliorating bleeding, tissue-repairing agents, remineralizing agents, and agents for suppressing hypersensitivity.

Specific examples of the medicinal ingredients include the following bactericides: anionic bactericides such as sodium cocoyl sarcosinate and sorbic acid; cationic bactericides such as chlorhexidine hydrochloride, chlorhexidine gluconate, benzalkonium chloride, and benzethonium chloride; amphoteric bactericides such as dodecyldiaminoethyl glycine; phenolic bactericides such as halogenated diphenyl ethers (e.g., triclosan (2',4,4'-trichloro-2-hydroxydiphenyl ether)) and isopropyl methylphenol; and hinokitiol.

Examples of circulation promoters include vitamin E compounds such as dl-$\alpha$-tocopheryl acetate, tocopherol succinate, and tocopheryl nicotinate; and enzymes such as dextranase, amylase, protease, mutanase, lysozyme, and lytic enzymes.

Examples of anti-inflammatory agents include epsilon-aminocaproic acid, dipotassium glycyrrhizinate, and $\beta$-glycyrrhetinic acid.

Examples of agents for ameliorating bleeding include tranexamic acid and ascorbic acid.

Examples of tissue-repair agents include allantoin.

Examples of remineralizing agents include fluoride compounds such as sodium fluoride.

Examples of agents for suppressing hypersensitivity include nitrates (potassium nitrate), aluminum lactate, hydroxyapatite, stannous fluoride, and arginine.

Other examples include plant extracts extracted with a water-soluble solvent, chlorophyll, sodium chloride, zinc chloride, and potassium nitrate.

Specific examples of surfactants include nonionic surfactants, anionic surfactants, cationic surfactants, and amphoteric surfactants.

Specific examples of nonionic surfactants include sugar fatty acid esters such as sucrose fatty acid esters and maltose fatty acid esters; sugar alcohol fatty acid esters such as maltitol fatty acid esters; sorbitan fatty acid esters such as sorbitan stearate and sorbitan monolaurate; polyoxyethylene sorbitan fatty acid esters such as polyoxyethylene sorbitan monolaurate (also referred to as polysorbate 20), polyoxyethylene sorbitan monostearate (also referred to as polysorbate 60), and polyoxyethylene sorbitan monooleate (also referred to as polysorbate 80); fatty acid alkanolamides such as lauric acid diethanolamide; polyoxyethylene alkyl ethers such as polyoxyethylene stearyl ether and polyoxyethylene oleyl ether; polyethylene glycol fatty acid esters such as polyethylene glycol monooleate and polyethylene glycol monolaurate; alkyl glycosides such as lauryl glycoside and decyl glycoside; polyglycerin fatty acid esters; polyoxyethylene glycerin fatty acid esters; polyoxyethylene fatty acid esters; alkyl glucosides; polyoxyethylene hydrogenated castor oil having an average number of added moles of ethylene oxide of 10, 20, 40, or 60; glycerin fatty acid esters; and polyoxyethylene-polypropylene block copolymers.

Specific examples of anionic surfactants include sulfate ester salts such as sodium lauryl sulfate and sodium polyoxyethylene lauryl ether sulfate; sulfosuccinate salts such as sodium lauryl sulfosuccinate and sodium polyoxyethylene lauryl ether sulfosuccinate; acylamino acid salts such as sodium cocoyl sarcosinate and sodium lauroyl methylalaninate; and sodium cocoyl methyl taurate.

Specific examples of cationic surfactants include PCA ethyl cocoyl arginate; quaternary alkylammonium salts such as cetyltrimethylammonium chloride, distearyldimethylammonium chloride, stearyldimethylbenzylammonium chloride, and stearyltrimethylammonium chloride; and chlorhexidine gluconate.

Specific examples of amphoteric surfactants include amino acid-type amphoteric surfactants such as N-lauryldiaminoethyl glycine and N-myristyldiethyl glycine; and betaine-type amphoteric surfactants such as alkyldimethylaminoacetate betaine, N-alkyl-N'-carboxymethyl-N'-hydroxyethyl ethylenediamine salts, 2-alkyl-N-carboxymethyl-N-hydroxyethyl imidazolinium betaine, and cocamidopropyl betaine.

Specific examples of abrasives include calcium carbonate, calcium phosphate, dibasic calcium phosphate, calcium pyrophosphate, insoluble sodium metaphosphate, titanium oxide, amorphous silica, crystalline silica, polishing silica, thickening silica, aluminosilicate, aluminum oxide, titanium oxide, aluminum hydroxide, and resins. The silica described above is also referred to as silicic anhydride.

Specific examples of humectants include propylene glycol, glycerin, sorbitol, polyethylene glycol, 1,3-butylene glycol, water, and alcohol.

Specific examples of thickeners include sodium polyacrylate, carrageenan, sodium carboxymethyl cellulose, sodium alginate, xanthan gum, hydroxyethyl cellulose, crystalline cellulose, hydroxypropyl methylcellulose, methylcellu-

lose, and propylene glycol alginate. Thickeners are also referred to as binders.

Specific examples of stabilizers include sodium edetate, sodium thiosulfate, sodium sulfite, calcium lactate, lanolin, triacetin, castor oil, and magnesium sulfate.

Specific examples of preservatives include 1,2-dibromo-2,4-dicyanobutane, photosensitizers, isothiazolone derivatives, hydantoin derivatives, parabens, sodium benzoate, and phenols.

Specific examples of sweeteners include saccharin, saccharin sodium, acesulfame potassium, stevia extract, palatinose, palatinit, erythritol, maltitol, xylitol, and lactitol.

Specific examples of pH adjusters include citric acid, phosphoric acid, malic acid, pyrophosphoric acid, lactic acid, tartaric acid, glycerophosphoric acid, acetic acid, nitric acid, chemically possible salts thereof, and sodium hydroxide.

Specific examples of antioxidants include tocopherols, dibutylhydroxytoluene, butylhydroxyanisole, and gallic acid esters.

The flavoring agent may be a natural flavoring agent or a synthetic flavoring agent. The flavoring agent may be a single flavoring agent or a blended flavoring agent.

Specific examples of flavoring agents include l-menthol, d-carvone, anethole, eugenol, methyl salicylate, limonene, ocimene, n-decyl alcohol, citronellol, $\alpha$-terpineol, methyl acetate, citronellyl acetate, methyl eugenol, cineole, linalool, ethyl linalool, thymol, spearmint oil, peppermint oil, lemon oil, orange oil, sage oil, rosemary oil, perilla oil, wintergreen oil, clove oil, eucalyptus oil, pimento oil, d-camphor, d-borneol, fennel oil, cinnamon oil, cinnamaldehyde, cornmint oil, and vanillin.

Specific examples of colorants include certified colors such as Green No. 1, Green No. 3, Blue No. 1, Yellow No. 4, Yellow No. 5, Red No. 102, and Red No. 3; sodium copper chlorophyllin; and titanium dioxide.

<Applied Forms, Dosage Forms, and Uses of Dentin Protective Agent and Oral Composition>

The applied form of the dentin protective agent and oral composition is not particularly limited, and the dentin protective agent and oral composition can be used, for example, as pharmaceuticals, quasi-drugs, cosmetics, or foods.

The dosage form of the dentin protective agent and oral composition is not particularly limited, and may be, for example, an ointment, paste, spray, gel, liquid, suspension, or gum containing a solvent such as water, alcohol, or a hydrocarbon.

The type of water used as the solvent is not particularly limited, and, for example, distilled water, pure water, ultrapure water, purified water, tap water, and the like may be used. The type of alcohol used as the solvent is not particularly limited, and, for example, ethanol may be used. Water and alcohol may also be used in combination. The type of hydrocarbon used as the solvent is not particularly limited, and, for example, gelled hydrocarbon, paraffin, squalane, and the like may be used.

Known uses may be appropriately adopted for the dentin protective agent and the oral composition. Specifically, examples include chewing preparations, orally dissolving preparations, orally disintegrating preparations, tongue-care agents, breath fresheners, toothpastes, mouthwashes, gargles, liquid dentifrices, biofilm dispersants, agents for preventing halitosis, gum massage agents, oral moistening agents, tongue-coating removers, oral preparations for topical application, oral bactericides, throat bactericides, oral and throat preparations, therapeutic agents for periodontal disease, denture adhesives, denture coating agents, denture stabilizers, denture preservatives, denture cleansers, and implant-care agents.

<Effects of the Present Embodiment>

The dentin protective agent and the oral composition exhibit an excellent dentin-protective effect due to the above components. More specifically, the caries-preventive effect can be improved by improving the demineralization suppression rate of dentin. In addition, the effect of preventing hypersensitivity is improved by improving the dentinal tubule occlusion rate.

<Advantages of the Present Embodiment>

The advantages of the dentin protective agent and oral composition of the present embodiment will now be described.

(1) The dentin protective agent and the oral composition of the present embodiment contain, as a calcium silicate raw material, calcium silicate having a Si/Ca ratio of 1.15 to 2.3, inclusive. Accordingly, the dentin-protective effect is improved. As a result, an effect of improving the acid resistance of dentin, a caries-preventive effect, and a hypersensitivity-preventive effect are obtained. In particular, elderly persons are prevented from suffering from root caries on dentin exposed by gingival recession, hypersensitivity, and the like.

(2) Conventionally, fluoride application has generally been used for the prevention of dental caries and hypersensitivity; however, due to the structural characteristics of dentin, dentin is less responsive to fluoride, and sufficient effects could not be obtained. The dentin protective agent and the oral composition of the present embodiment improve the caries-preventive effect and the hypersensitivity-preventive effect without relying on fluoride.

<Modifications of the Present Embodiment>

The above embodiment may be modified as follows. The present embodiment and the following modifications may be implemented in combination with each other as far as no technical inconsistency arises.

- The dentin protective agent and oral composition of the present embodiment may be applied to kept animals such as pets and livestock, other than humans.

Examples

Examples and the like will be given below in order to describe the configuration and effects of the present disclosure more specifically, but the present disclosure is not limited to these Examples.

Dentin protective agents of Examples 1 and 2 and Comparative Examples 1 to 3 shown in Table 2 were produced in accordance with conventional methods. Specifically, calcium silicate having the Si/Ca ratios shown in Table 2 was dissolved in water to a concentration of 10 mass% to prepare aqueous solutions as dentin protective agents. The Si/Ca ratios of the calcium silicates shown in Table 2 were measured by the above energy-dispersive X-ray spectroscopy (SEM-EDX), and are average values obtained by measuring each sample four times. For reference, for the Si/Ca ratios of the calcium silicates used in Examples 1 and 2, the maximum and minimum values of the measured values are shown.

(Evaluation Test)

For the dentin protective agents of Examples 1 and 2 and Comparative Examples 1 to 3, the dentin-protective effect was evaluated by measuring dentinal tubule occluding ability and demineralization suppressing ability according to the methods described below. The evaluation methods and results are shown below.

(Brushing Treatment and Dentinal Tubule Occlusion Test)

(1) Dentin is cut out from the root surface portion of an extracted bovine tooth and embedded in a resin (polymethyl methacrylate). The embedded dentin is then polished with waterproof abrasive paper to expose the surface, thereby preparing a bovine tooth block.
(2) The bovine tooth block is immersed in a 2% (w/w) aqueous citric acid solution for 2 minutes.
(3) After immersion in distilled water, ultrasonic treatment is performed.
(4) A part of the tooth surface is coated with varnish to provide a sound surface.
(5) Brushing is performed for 30 seconds in a sample solution (*1) using a brushing machine.

*1: Sample solution (aqueous solution containing 10% (w/w) calcium silicate, 0.5% (w/w) sodium carboxymethyl cellulose, and 10% (w/w) glycerin)

(6) The surface is lightly washed with distilled water and immersed in artificial saliva (*2) for 8 hours at 37°C.

*2: Artificial saliva ($CaCl_2$: 1.5 mM, $KH_2PO_4$: 0.9 mM, KCl: 130 mM, HEPES: 20 mM, pH 7.0 (KOH))

(7) The surface is washed with distilled water.
(8) Brushing is performed for 30 seconds in the sample solution using the brushing machine.
(9) The surface is lightly washed with distilled water and immersed in artificial saliva for 16 hours at 37°C.
(10) The surface is washed with distilled water.
(The above (5) to (10) are repeated twice.)
(11) After removal of the varnish, the surface of each sample is observed at the same magnification using a laser microscope, and the number of unoccluded dentinal tubules is determined. The dentinal tubule occlusion rate is calculated according to the following equation.

Dentinal tubule occlusion rate (%) = {[number of dentinal tubules (sound surface)] - [number of dentinal tubules (treated surface)] / [number of dentinal tubules (sound surface)]} × 100

The effectiveness of dentinal tubule occlusion was evaluated according to the following criteria. The results are shown in Table 2.

3: dentinal tubule occlusion rate is greater than 70%
2: dentinal tubule occlusion rate is greater than 50% and 70% or less
1: dentinal tubule occlusion rate is 50% or less

(Demineralization Suppression Test)

Samples after the brushing treatment in the dentinal tubule occlusion test were used.

(1) A part of the tooth surface is coated with varnish to provide a sound surface.
(2) The sample is immersed in a demineralizing solution (0.87 M aqueous citric acid solution, pH 1.36) for 2 hours.
(3) After removal of the varnish, the surface is lightly washed with distilled water, and the step relative to the reference surface is measured with a laser microscope. The demineralization suppression rate is calculated from the following equation.

Demineralization suppression rate (%) = {[step (sound surface)] - [step (treated surface)] / [step (sound surface)]} × 100

The effectiveness of demineralization suppression was evaluated according to the following criteria. The results are shown in Table 2.

3: demineralization suppression rate is greater than 70%
2: demineralization suppression rate is greater than 50% and 70% or less
1: demineralization suppression rate is 50% or less

[Table 2]

| | Calcium Silicate Si/Ca Ratio (min-max) | Demineralization Suppression Rate (%) | Effectiveness | Dentinal Tubule Occlusion Rate (%) | Effectiveness |
|---|---|---|---|---|---|
| Example 1 | 1.68 (1.51-1.92) | 89.5 | 3 | 92 | 3 |
| Example 2 | 1.37 (1.18-1.49) | 84.35 | 3 | 97.8 | 3 |
| Comparative Example 1 | 6.64 | 52.27 | 2 | 48.6 | 1 |
| Comparative Example 2 | 2.59 | 41.61 | 1 | -24.4 | 1 |
| Comparative Example 3 | 1.02 | 21.25 | 1 | 0.56 | 1 |

(Evaluation Results)

As shown in Table 2, in each Comparative Example, the dentinal tubule occlusion rate was less than 50%. In contrast, in each Example, the dentinal tubule occlusion rate was greater than 50%. In addition, in each Example, the demineralization suppression rate was greater than 70%. Accordingly, it was confirmed that the configurations of each Example had a particularly excellent dentin-protective effect.

(Clauses)

Technical ideas that can be understood from the above embodiment and modification will now be described.

(a) An oral composition or dentin protective agent, including calcium silicate having a Si/Ca ratio of 1.2 to 2.2, inclusive.
(b) The oral composition or dentin protective agent for use in preventing dentin caries or hypersensitivity.

## Claims

1. An oral composition, comprising calcium silicate having a Si/Ca ratio of 1.15 to 2.3, inclusive.

2. The oral composition according to claim 1, wherein the oral composition is for dentin protection.

3. A dentin protective agent, comprising calcium silicate having a Si/Ca ratio of 1.15 to 2.3, inclusive.

# INTERNATIONAL SEARCH REPORT

International application No. **PCT/JP2024/042346**

**A. CLASSIFICATION OF SUBJECT MATTER**

***A61K 8/25***(2006.01)i; ***A61K 33/06***(2006.01)i; ***A61P 1/02***(2006.01)i; ***A61Q 11/00***(2006.01)i
FI: A61K8/25; A61P1/02; A61Q11/00; A61K33/06

According to International Patent Classification (IPC) or to both national classification and IPC

**B. FIELDS SEARCHED**

Minimum documentation searched (classification system followed by classification symbols)

A61K8/25; A61K33/06; A61P1/02; A61Q11/00

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2010-503614 A (UNILEVER NV) 04 February 2010 (2010-02-04) claims, paragraph [0045], examples | 1-3 |
| X | JP 2016-529300 A (UNILEVER NV) 23 September 2016 (2016-09-23) claims, paragraphs [0001], [0043], examples | 1-3 |
| A | JP 2013-544883 A (COLGATE-PALMOLIVE COMPANY) 19 December 2013 (2013-12-19) claims, examples | 1-3 |
| A | JP 10-120540 A (SUNSTAR INC.) 12 May 1998 (1998-05-12) claims, examples | 1-3 |
| A | JP 10-139644 A (SUNSTAR INC.) 26 May 1998 (1998-05-26) claims, examples | 1-3 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 January 2025** | **18 February 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/042346**

| Patent document cited in search report | Publication date (day/month/year) | Patent family member(s) | Publication date (day/month/year) |
|---|---|---|---|
| JP 2010-503614 A | 04 February 2010 | US 2009/0319052 A1<br>claims, paragraph [0046], examples<br>WO 2008/015117 A2<br>EP 2046405 A2<br>CN 101528279 A | |
| JP 2016-529300 A | 23 September 2016 | US 2016/0206525 A1<br>claims, paragraphs [0001], [0063], examples<br>WO 2015/036277 A1<br>EP 3043868 A1<br>CN 105517633 A | |
| JP 2013-544883 A | 19 December 2013 | US 2013/0251772 A1<br>claims, examples<br>WO 2012/078136 A1<br>EP 2648677 A1<br>CN 103228246 A | |
| JP 10-120540 A | 12 May 1998 | (Family: none) | |
| JP 10-139644 A | 26 May 1998 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H11228368 A